# EUROPEAN PATENT SPECIFICATION

(11) **EP 3 003 400 B1**
(45) Date of publication and mention of the grant of the patent: **12.09.2018**
(21) Application number: 14742390.9
(22) Date of filing: 05.06.2014
(51) Int. Cl.: A61K 51/08, A61P 19/02, A61K 9/00

(54) **TREATMENT OF IMMUNE, INFLAMMATORY AND DEGENERATIVE ARTHRITIDES WITH TIN-117M**
BEHANDLUNG VON IMMUN-, ENTZÜNDLICHEN UND DEGENERATIVEN ARTHRITIDEN MIT ZINN-117M
TRAITEMENT D'ARTHRITES AUTO-IMMUNES, INFLAMMATOIRES ET DÉGÉNÉRATIVES AVEC L'ETAIN-117M

(30) Priority: 05.06.2013 US 201361831177 P
(43) Date of publication of application: 13.04.2016
(73) Proprietor: Serene, LLC, The Woodlands, TX 77380 (US)
(72) Inventor: GONZALES, Gilbert, R., Tucson, AZ 85745 (US); STEVENSON, Nigel, R., Sugar Hill, GA 30518 (US)
(74) Representative: Findlay, Alice Rosemary
(86) International application number: PCT/US2014/041065
(87) International publication number: WO 2014/197681

(56) References cited:
- WO-A1-2004/052408
- WO-A1-2009/070787
- WO-A2-2010/064012
- US-A1- 2010 204 457
- US-B1- 6 231 832
- SRIVASTAVA SURESH C: "The role of electron-emitting radiopharmaceuticals in the palliative treatment of metastatic bone pain and for radiosynovectomy: Applications of conversion electron emitter tin-117m", BRAZILIAN ARCHIVES OF BIOLOGY AND TECHNOLOGY, INSTITUTO DE TECNOLOGIA DO PARANA, BR , vol. 50, no. Special number SI 1 September 2007 (2007-09-01), pages 49-62, XP008171226, ISSN: 1516-8913 Retrieved from the Internet: URL:http://www.scielo.br/pdf/babt/v50nspe/ a07v50ns.pdf
- STEVENSON NIGEL ET AL: "Production and applications of very high specific activity Sn-117m and labeled chelates/molecules", JOURNAL OF LABELLED COMPOUNDS AND RADIOPHARMACEUTICALS, vol. 56, no. Suppl. 1, May 2013 (2013-05), page S40, XP008171213, & 20TH INTERNATIONAL SYMPOSIUM ON RADIOPHARMACEUTICAL SCIENCES; JEJU, SOUTH KOREA; MAY 12 -17, 2013
- SIMON JAIME ET AL: "Sn-117m labeled annexin for vulnerable plaque", AMERICAN CHEMICAL SOCIETY. ABSTRACTS OF PAPERS (AT THE NATIONAL MEETING), AMERICAN CHEMICAL SOCIETY, US , vol. 245 1 April 2013 (2013-04-01), page 10, XP008171214, ISSN: 0065-7727 Retrieved from the Internet: URL:http://abstracts.acs.org/chem/245nm/pr ogram/view.php?obj_id=185755&terms=
- WILBUR D ET AL: "MICROWAVE ASSISTED CHELATION OF SN-117M WITH A SULFHYDRYL-REACTIVE DOTA DERIVATIVE FOR LABELING OF ANNEXIN V", JOURNAL OF LABELLED COMPOUNDS AND RADIOPHARMACEUTICALS, JOHN WILEY, CHICHESTER; GB , vol. 52, no. Supplement 1 1 January 2009 (2009-01-01), page S443, XP008171217, ISSN: 0362-4803, DOI: 10.1002/JLCR.1649 Retrieved from the Internet: URL:http://onlinelibrary.wiley.com/doi/10. 1002/jlcr.1649/pdf
- SAVIO EDUARDO ET AL: "188Re radiopharmaceuticals for radiosynovectomy: evaluation and comparison of tin colloid, hydroxyapatite and tin-ferric hydroxide macroaggregates", BMC NUCLEAR MEDICINE, BIOMED CENTRAL, LONDON, GB, vol. 4, no. 1, 30 January 2004 (2004-01-30), page 1, XP021004438, ISSN: 1471-2385, DOI: 10.1186/1471-2385-4-1
- PONSARD B ET AL: "Production of Sn-117m in the BR2 high-flux reactor", APPLIED RADIATION AND ISOTOPES, ELSEVIER, OXFORD, GB, vol. 67, no. 7-8, 1 July 2009 (2009-07-01) , pages 1158-1161, XP026148757, ISSN: 0969-8043, DOI: 10.1016/J.APRADISO.2009.02.023 [retrieved on 2009-02-20]

## Description

### RELATED APPLICATION

The present application claims priority to U.S. Serial No. 61/831,177, filed June 5, 2013.

### BACKGROUND OF THE INVENTION

Radiosynovectomy (RSV) is a local intra-articular injection of radionuclides generally in colloidal form for treatment of rheumatoid arthritis and hemophiliac arthritis in man. Evidence in animals also indicate a positive effect (treatment) for degenerative and osteoarthritis by this same method. Typically, RSV employs Y-90, P-32, Rel86, Re-188 and Er-169. These all are beta-emitting isotopes with a half life bearing from 2.7 to 14.3 days, and an average penetration of 0.3 to 3.6 millimeters. Beta-emitting isotopes may not be ideal for such treatment, but they are effective in certain applications. SRIVASTAVA SURESH C. et al, BRAZILIAN ARCHIVES OF BIOLOGY AND TECHNOLOGY, INSTITUTO DE TECNOLOGIA DO PARANA, BR,vol. 50, no. Special number SI 1 September 2007 (2007-09-01), pages 49-62, teaches the use of colloidal radiopharmaceuticals for radiosynovectomy in the treatment of rheumatoid arthritis. The compounds are injected intra-articular. It is mentioned that a study was initiated to assess the efficacy of Sn-117m DTPA in radiosynovectomy after intra-articular administration in patients with rheumatoid arthritis.

### SUMMARY OF THE INVENTION

The present invention is premised on the realization that immune, inflammatory and degenerative arthritides can be treated by intra-articular injection of high specific activity tin-117m compounds as defined in the claims. In particular, tin-117m colloidal compounds can be injected for treatment of rheumatoid arthritis, hemophiliac arthropathy and osteoarthritis. The present application relates to Tin-117m, having a specific activity of > 100 Ci/g, for use in treating immune, inflammatory and degenerative arthritides, wherein said treating comprises injecting the tin-117m intra-articularly into a joint in humans or animals.

In a further improvement of the present invention, tin-117 annexin compounds can be injected intra-articularly or systemically for treatment of rheumatoid arthritis, hemophiliac arthropathy and osteoarthritis. The tin-117m accumulates in the affected tissue providing a strong radiation dose from conversion electrons, which travels a very precise distance. The radiation has an effective distance of 290 microns (depending on the density of the tissue traversed), minimizing damaging nearby cells.

The present invention will be further appreciated in light of the following detailed description and drawings in which:

### BRIEF DESCRIPTION OF THE DRAWINGS

FIG. 1 is a graph showing the average ankle measurements of subjects in the second study over time. Ankle size is a measure of the effectiveness of the collagen induced "rheumatoid arthritis-like" model;

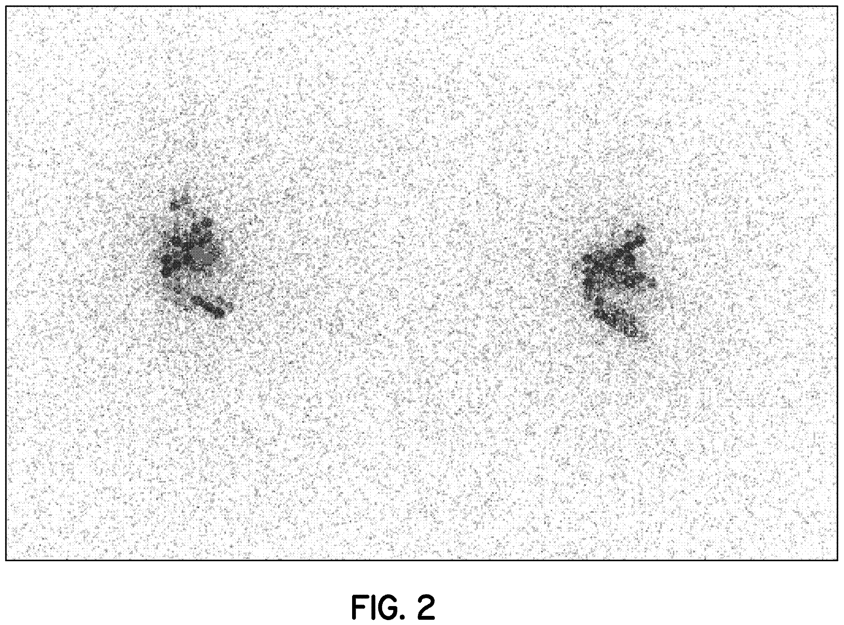

FIG. 2 is an autoradiograph showing tin-117m colloid injected in a subject in the first study. In this example, "honeycomb" collimators were inserted between the sample and detector in order to provide superior resolution images;

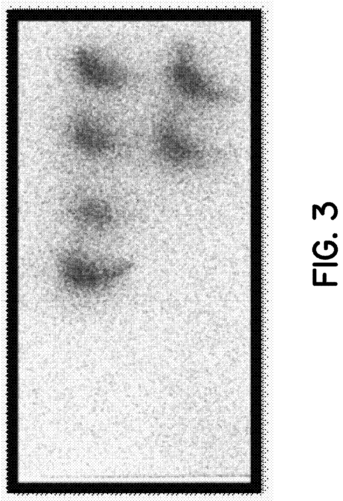

FIGS. 3 are autoradiographs of test subjects taken and showing tin-117m annexin in test subjects in the second study. In this example, several animal joints were simultaneously viewed;

### DETAILED DESCRIPTION

The present application relates to tin-117m, having a specific activity of > 100 Ci/g, for use in treating immune, inflammatory and degenerative arthritides, wherein said treating comprises injecting the tin-117m intra-articularly into a joint in humans or animals. The invention is defined by the claims. Any subject-matter falling outside the scope of the claims is provided for information purposes only. Tin-117m is injected into the synovium of an affected joint to treat rheumatoid arthritis, hemophiliac arthropathy and osteoarthritis by radiosynovectomy.

Tin-117m annexin is formed from a high specific activity (HSA) no-carrier-added tin-117m (> 100 Ci/g). There are various methods to produce no-carrier added tin-117m. One such method is disclosed in Stevenson U.S. Patent 8,257,681, although other methods of forming the tin-117m could be employed in the present invention. Tin-117-m is a unique radioisotope. Although it emits some gamma radiation, the primary source of the therapeutic radiation is conversion electrons. The radiation from the conversion electron penetrates up to 290 microns, and, therefore, does not damage cells any farther than 290 microns away from the tin atom. This allows for the use of higher doses since the distant radio-sensitive regions such as bone marrow will be spared.

Tin-117m may be bonded to an annexin molecule. Annexins are a class of molecule having the ability to bind with high affinity to membrane lipids in the presence of millimolar concentrations of calcium. There are several different annexins. The term "annexin" includes native annexin purified from natural sources, such as human placenta or annexin molecules containing a native sequence produced through, for example, genetic engineering, or other means. The term annexin unless otherwise specified, includes annexins derived from or produced by any source.

Typically a linking molecule is used to attach the tin-117m annexin. One such linking molecule is 1,4,7,10tetraazacylododecane - 1,4,7,10 - tetra acetic acid, or DOTA. Usually, aminobenzyl DOTA is used. Formation of this molecule is disclosed in U.S. Patent No. 8,283,167.

According to the present invention, the tin-117m annexin V in a suitable sterile carrier, such as saline, is injected directly into the affected joint or is systemically injected to treat multiple joints. Annexin V is known to bind to phosphatidyl serine which is exposed in cells during apoptosis. Recent work in the imaging and treatment of vulnerable or unstable plaque provided strong evidence that very low doses of tin-117m annexin were able to have positive effects. The data supports the observation that the tin-117m annexin is inducing apoptosis even at these "hormesis" doses which allows for treating various arthritides with low quantities of tin-117m. The dosage may vary widely depending on the particular joint, as well as the extent of the damage. Generally, 0.1 milliCuries to 20 milliCuries will be injected, or, more particularly, 0.1 milliCuries to 5 milliCuries According to one embodiment of the present invention, a dosage of HSA tin-117m is sufficient to provide conversion electron therapy which is hermetic. A much lower dosage provides hormesis. Generally, 0.01 to <0.1 milliCuries can be typically used for hormesis doses. These injections may be repeated after a period of time, and, further, gamma emissions from the injected tin-117m can be imaged, if desired.

According to a second aspect of the present invention, a tin-117m colloidal solution is injected into an affected joint, again, in an RSV procedure. Any colloid typically used for RSV purposes can be used in the present invention. Typical particles include citrate/silicate; colloid/sulfide, hydroxide and citrate. These are injected intra-articularly in the same manner as the tin-117m annexin V, and, further, in similar dosage. Typical particle sizes range from 0.1-20 µm, or more particularly, 3-20 µm.

Of particular note in this invention is the ability to use known cold (non-radioactive) tin colloids that are subsequently coated with HSA Sn-117m. This lends itself to a kit preparation in which a radiopharmacy would maintain colloidal cold tin. The HSA tin-117m would be sent to the pharmacy where it would be mixed with the cold tin and provided to the doctor or veterinarian for injection.

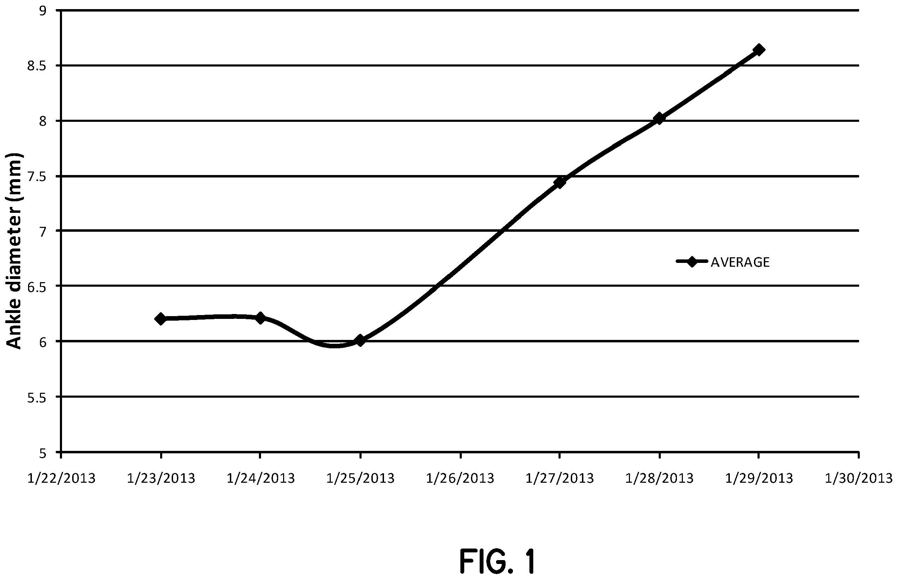

In order to test the efficacy of the present invention, a standard arthritic rat model was employed. Specifically, rodents were injected with collagen, which induced a reaction similar to rheumatoid arthritis. Figure 1 shows how the model is validated by measurements of ankle size that increase after collagen injections. Two different studies were conducted. Table 1 shows the experiment design for the first study.

### Experimental Design - Study 1

### Schedule for Inducing Arthritis and Dosing with Sn-117m COLLOID

**Table 1 Experimental Design for HSA Sn-117m Colloid Study**

| | Day 0 | Day 1 | Day 2 | Day 3 | Day 4 | Day 5 | Day 6 | Day 7 | Day 8 | Day 9 | Day 10 | Day 11 | Day 12 | Day 13 | Day 14 | Day 15 |
|---|---|---|---|---|---|---|---|---|---|---|---|---|---|---|---|---|
| | 11-Dec | 12-Dec | 13-Dec | 14-Dec | 15-Dec | 16-Dec | 17-Dec | 18-Dec | 19-Dec | 20-Dec | 21-Dec | 22-Dec | 23-Dec | 24-Dec | 25-Dec | 26-Dec |
| Rat 1 | a | | | | | | | b w | + | + | + | + | + | + | + | q + |
| Rat 2 | a | | | | | | | b w | + | + | + | + | + | + | + | q + |
| Rat 3 | a | | | | | | | b w | + | x+ | + | + | + | + | + | q + |
| Rat 4 | a | | | | | | | b w | + | x+ | + | + | + | + | + | q + |
| Rat 5 | a | | | | | | | by | + | z+ | + | + | + | + | + | q+ |
| Rat 6 | a | | | | | | | by | + | + | + | + | + | + | + | q+ |

| | | | | | | | | | | | | | | | | |
|---|---|---|---|---|---|---|---|---|---|---|---|---|---|---|---|---|
| a - Inject each rat with 200uL of collagen on the left and 200uL on the right side. b - Booster injection 100uL of collagen between the two previous injection sites. w - Dose with ∼8.3uCi Sn-117m colloid x- Dose with ∼33.2uCi Sn-117m colloid y - Dose with ∼8.3uCi Sn-n7m Homogenous Precipitation z - Dose with ∼33.2uCi Sn-117m Homogenous Precipitation + - Measure ankles with calipers and record the information. q - Sacrifice and perform full biodistribution including removing thymus, feet and legs. Place thymus, liver, spleen, feet and legs into 10% formalin | | | | | | | | | | | | | | | | |

200 micro liters of collagen were injected on the left and right knees at day zero. At day seven, booster injections of 100 micro liters of collagen were again injected and also 8.3 µCi of tin-117m colloid. Two days later an additional 33.2 µCi of tin-117m colloid was added to half of the animals for a total dose of 41.5 µCi.

### Experimental Design - Study 2

### Schedule for Inducing Arthritis and Dosing with Sn-117m ANNEXIN

**Table 2 Experimental Design for HSA Sn-117m Annexin Study**

| | Day 0 | Day 1 | Day 2 | Day 3 | Day 4 | Day 5 | Day 6 | Day 7 | Day 8 | Day 9 | Day 10 | Day 11 | Day 12 | Day 13 | Day 14 |
|---|---|---|---|---|---|---|---|---|---|---|---|---|---|---|---|
| | 15-Jan | 16-Jan | 17-Jan | 18-Jan | 19-Jan | 20-Jan | 21-Jan | 22-Jan | 23-Jan | 24-Jan | 25-Jan | 26-Jan | 27-Jan | 28-Jan | 29-Jan |
| Rat 1 | a | | | | | | | b | + | + | x+ | + | + | + | z+ |
| Rat 2 | a | | | | | | | b | + | + | x+ | + | + | + | z+ |
| Rat 3 | a | | | | | | | b | + | + | y+ | + | + | + | z+ |
| Rat 4 | a | | | | | | | b | + | + | y+ | + | + | + | z+ |

| | | | | | | | | | | | | | | | |
|---|---|---|---|---|---|---|---|---|---|---|---|---|---|---|---|
| a - Inject each rat with 200uL of collagen on the left and 200uL on the right side. b - Booster injection 100uL of collagen between the two previous injection sites x- Dose with ∼50uCi Sn-117m Annexin (retro-orbital) y- Dose with ∼50uCi Sn-117m Annexin (Synovium) + - Measure ankles with calipers and record the information. z - Sacrifice and perform full biodistribution including removing thymus, feet and legs. Place feet and legs into 10% formalin. | | | | | | | | | | | | | | | |

Table 2 shows the similar rat study, but 50 µCi of tin-117m-DOTA annexin was either directly injected into the synovium, or retro-orbitally to determine systemic efficacy. At around day 14, the animals were sacrificed and tissues sent for histological analyses.

### 1^{st} Study - Biodistribution

**Table 3 Biodistribution Results for HSA Sn-117m Colloid Study**

| **Animal** | **Injection Location** | **Dose (mCi) (St/Ho)** | **Left leg (% ID)** | **Right Leg (% ID)** |
|---|---|---|---|---|
| 1 | L Synovium | 8.3 (St) | 65.9 | 2.3 |
| 2 | L Synovium | 8.3 (St) | 62.8 | 2.3 |
| 3 | L Synovium | 41.5 (St) | 68.2 | 2.0 |
| 4 | L Synovium | 41.5 (St) | 68.7 | 2.0 |
| 5 | L Synovium | 41.5 (Ho) | 98.7 | 0.0 |
| 6 | L Synovium | 8.3 (Ho) | 99.9 | 0.0 |

### 2^{nd} Study - Biodistribution

**Table 4 Biodistribution Results for HSA Sn-117m Annexin Study**

| **Animal** | **Injection Location** | **Dose (mCi)** | **Left leg (% ID)** | **Right Leg (% ID)** |
|---|---|---|---|---|
| 1 | Retro-orbital | 50 | 0.1 | 0.1 |
| 2 | Retro-orbital | 50 | 0.1 | 0.1 |
| 3 | L Synovium | 50 | 20.1 | 0.1 |
| 4 | L Synovium | 50 | 7.3 | 0.1 |

### Histopathology - 1^{st} Study (Knees)

**Table 5 Histopathology Results for HSA Sn-117m Colloid Study**

| Treatment | | Species: | | Rat | | |
|---|---|---|---|---|---|---|
| Group | | Necropsy Day: | | X | | |
| Group 1 | | Histopathology Scores (0-5, 0=normal, 5=severe) | | | | Summed |
| N/A | | Inflammation | Pannus | Cartilage Damage | Bone Resorption | Histopathology |
| 0 | | Score | Score | Score | Score | Scores |
| 1 | R | 4 | 0.5 | 0.5 | 0 | 5 |
| | L | 4 | 0.5 | 1 | 0.5 | 6 |
| 2 | R | 2 | 0.5 | 1 | 0 | 3.5 |
| | L | 5 | 1 | 1 | 1 | 8 |
| 3 | R | 2 | 0.5 | 1 | 0 | 3.5 |
| | L | 4 | 1 | 1 | 0 | 6 |
| 4 | R | 4 | 1 | 1 | 1 | 7 |
| | L | 4 | 1 | 1 | 0.5 | 6.5 |
| 5 | R | 5 | 2 | 2 | 2 | 11 |
| | L | 5 | 1 | 2 | 1 | 9 |
| 6 | R | 5 | 1 | 1 | 1 | 8 |
| | L | 5 | 1 | 2 | 1 | 9 |
| Mean | | 4.08 | 0.92 | 1.21 | 0.67 | 6.88 |
| SE | | 0.31 | 0.12 | 0.14 | 0.18 | 0.65 |
| ANOVA Results (grp 2) | | #REF! | #REF! | #REF! | #REF! | #REF! |
| % Inhibition to G2 | | #REF! | #REF! | #REF! | #REF! | #REF! |

### Histopathology - 2^{nd} Study (Knees)

**Table 6 Histopathology Results for HSA Sn-117m Annexin Study**

| Treatment | | Species: | Rat | | | |
|---|---|---|---|---|---|---|
| Group | | Necropsy Day: | X | | | |
| Group 1 | | Histopathology Scores (0-5, 0=normal, 5=severe) | | | | Summed |
| 0 | | Inflammation | Pannus | Cartilage Damage | Bone Resorption | Histopathology |
| 0 | | | | | | Scores |
| 1 | R | 0.5 | 0.5 | 0.5 | 0 | 1.5 |
| | L | 5 | 1 | 1 | 1 | 8 |
| 2 | R | 4 | 0.5 | 0.5 | 0.5 | 5.5 |
| | L | 3 | 0.5 | 0.5 | 0 | 4 |
| 3 | R | 5 | 1 | 2 | 1 | 9 |
| | L | 5 | 1 | 2 | 1 | 9 |
| 4 | R | 5 | 1 | 1 | 0.5 | 7.5 |
| | L | 5 | 1 | 2 | 1 | 9 |
| Mean | | 4.04 | 0.92 | 1.33 | 0.75 | 7.04 |
| SE | | 0.48 | 0.12 | 0.21 | 0.17 | 0.89 |

Tables 3 and 4 show the biodistribution of the radioactive product at the end of the studies. This varies significantly depending on the colloid formulation or the mode of administration of the annexin product. However, very high retention is observed for some colloids in particular. Fi gures 2 and 3 show radiographs of the localized containment of the intra-articular injections for both studies.

Tables 5 and 6 show the hi stopathology results for the two studies. In the first study, synovial colloidal suspension injections of tin-117m appears to be effective and the product is uniformly distributed throughout the synovial joint cavity. Likewise, data from the second study suggests that the ti n-1 17-DOTA annexin appears to be effective when administered directly into the synovium. The study does serve to prove the validity of the model for this application and suggests the therapeutic effectiveness of Sn-117m.

This has been a description of the present invention along with the preferred method of practicing the present invention. However, the invention itself should only be defined by the appended claims, WHEREIN WE CLAIM:

## Claims

1. Tin-117m, having a specific activity of > 100 Ci/g, for use in treating immune, inflammatory and degenerative arthritides, wherein said treating comprises injecting the tin-117m intra-articularly into a joint in humans or animals.

2. Tin-117m for use according to claim 1 wherein said tin-117m is a tin-117m colloidal compound.

3. Tin-117m for use according to claim 2 wherein said colloidal compound is a non-radioactive tin colloid base that is coated with said tin-117m.

4. Tin-117m for use according to claim 1 wherein said treating comprises injecting 0.1 to 20 milliCuries of said tin-117m in each joint.

5. Tin-117m for use according to claim 1 wherein said treating comprises injecting 0.01 to <0.1 milliCuries of said tin-117m in a joint providing hormetic therapy.

6. Tin-117m for use according to claim 1 wherein said use further comprises combining said tin-117m with non-radioactive colloidal tin to form tin-117m coated colloidal tin and injecting said coated colloidal tin intra-articularly.

## Patentansprüche

1. Zinn-117m, welches eine spezifische Aktivität von > 100 Ci/g aufweist, zur Verwendung bei der Behandlung von immun-, entzündlichen und degenerativen Arthritiden, wobei die Behandlung das intraartikuläre Injizieren des Zinn-117m in ein Gelenk eines Menschen oder eines Tieres umfasst.

2. Zinn-117m zur Verwendung nach Anspruch 1, wobei Zinn-117m eine kolloidale Zinn-117m Verbindung ist.

3. Zinn-117m zur Verwendung nach Anspruch 2, wobei die kolloidale Verbindung eine nichtradioaktive Zinn-kolloidale Base ist, welche mit dem Zinn-117m beschichtet ist.

4. Zinn-117m zur Verwendung nach Anspruch 1, wobei die Behandlung das Injizieren von 0,1 bis 20 milliCuries des Zinn-117m in jedes Gelenk umfasst.

5. Zinn-117m zur Verwendung nach Anspruch 1, wobei die Behandlung das Injizieren von 0,01 bis < 0,1 milliCuries des Zinn-117m in ein Gelenk zum Bereitstellen einer Hormontherapie umfasst.

6. Zinn-117m zur Verwendung nach Anspruch 1, wobei die Verwendung ferner das Kombinieren des Zinn-117m mit nicht-radioaktivem kolloidalen Zinn, um mit Zinn-117m beschichtetes kolloidale Zinn zu bilden und das intraartikuläre Injizieren des beschichteten kolloidalen Zinns umfasst.

## Revendications

1. Étain-117m, ayant une activité spécifique > 100 Ci/g, pour une utilisation dans le traitement des arthrites immunes inflammatoires et dégénératives, où ledit traitement comprend l'injection de l'étain-117m par voie intra-articulaire dans une articulation chez des humains ou des animaux.

2. Étain-117m pour une utilisation selon la revendication 1, ledit étain-117m étant un composé colloïdal de l'étain-117m.

3. Étain-117m pour une utilisation selon la revendication 2, ledit composé colloïdal est à base d'étain colloïdal non radioactif revêtu avec ledit étain-117m.

4. Étain-117m pour une utilisation selon la revendication 1, où ledit traitement comprend l'injection de 0,1 à 20 milliCuries dudit étain-117m dans chaque articulation.

5. Étain-117m pour une utilisation selon la revendication 1, où ledit traitement comprend l'injection de 0,01 à < 0,1 milliCuries dudit étain-117m dans une articulation, assurant une thérapie hormétique.

6. Étain-117m pour une utilisation selon la revendication 1, où ladite utilisation comprend en outre la combinaison dudit étain-117m avec l'étain colloïdal non radioactif pour former de l'étain colloïdal revêtu d'étain-117m et l'injection par voie intra-articulaire dudit étain colloïdal revêtu.
